# EUROPEAN PATENT SPECIFICATION

(11) **EP 1 015 575 B1**
(45) Date of publication and mention of the grant of the patent: **19.05.2010**
(21) Application number: 98907912.4
(22) Date of filing: 18.03.1998
(51) Int. Cl.: C12N 15/10, C12Q 1/68

(54) **SHUFFLING OF HETEROLOGOUS DNA SEQUENCES**
VERMISCHEN VON HETEROLOGEN DNS-SEQUENZEN
REARRANGEMENT DE SEQUENCES D'ADN HETEROLOGUES

(30) Priority: 18.03.1997 DK 30497; 17.04.1997 DK 43297
(43) Date of publication of application: 05.07.2000
(73) Proprietor: Novozymes A/S, 2880 Bagsvaerd (DK)
(72) Inventor: BORCHERT, Torben, Vedel, DK-4040 Jyllinge (DK); KRETZSCHMAR, Titus, D-81739 Munich (DE); CHERRY, Joel, R., Davis, CA 95616 (US)
(86) International application number: PCT/DK1998/000105
(87) International publication number: WO 1998/041623

(56) References cited:
- WO-A-95/22625

## Description

### FIELD OF THE INVENTION

The present invention relates to a new method of shuffling especially heterologous polynucleotide sequences, screening and/or selection of new recombinant proteins resulting therefrom having a desired biological activity, and especially to the production and identification of novel proteases exhibiting desired properties.

### BACKGROUND OF THE INVENTION

It is generally found that a protein performing a certain bioactivity exhibits a certain variation between genera, and even between members of the same species differences may exist. This variation is even more outspoken at the genomic level.

This natural genetic diversity among genes coding for proteins having basically the same bioactivity has been generated in nature over billions of years and reflects a natural optimisation of the proteins coded for in respect of the environment of the organism in question.

However, in general it has been found that the naturally occurring bioactive molecules are not optimized for the various uses to which they are put by mankind, especially when they are used for industrial purposes.

It has therefore been of interest to industry to identify such bioactive proteins that exhibit optimal properties in respect of the use for which it is intended.

This has been done for many years by screening of natural sources, or by use of mutagenesis. For instance, within the technical field of enzymes for use in *e.g*. detergents, the washing and/or dishwashing performance of *e.g*. naturally occurring proteases, lipases, amylases and cellulases has been improved significantly by *in vitro* modifications of the enzymes.

In most cases these improvements have been obtained by site-directed mutagenesis resulting in substitution, deletion or insertion of specific amino acid residues which have been chosen either on the basis of their type or on the basis of their location in the secondary or tertiary structure of the mature enzyme (see for instance US patent no. 4,518,584).

### Prior Art:

Numerous methods to create genetic diversity, such as by site directed or random mutagenesis, have been proposed and described in scientific literature as well as patent applications. For further details in this respect reference is made to the related art section of WO 95/22625, wherein a review is provided.

One method of the shuffling of homologous DNA sequences has been described by Stemmer (Stemmer, (1994), Proc. Natl. Acad. Sci. USA, Vol. 91, 10747-10751; Stemmer, (1994), Nature, vol. 370, 389-391). The method concerns shuffling homologous DNA sequences by using *in vitro* PCR techniques. Positive recombinant genes containing shuffled DNA sequences are selected from a DNA library based on the improved function of the expressed proteins.

WO 95/22625 is believed to be the most pertinent reference in relation to the present invention in its "gene shuffling" aspect. In WO 95/22625 a method for shuffling of homologous DNA sequences is described. An important step in the method described in WO 95/22625 is to cleave the homologous template double-stranded polynucleotide into random fragments of a desired size followed by homologously reassembling of the fragments into full-length genes.

A disadvantage inherent to the method of WO 95/22625 is, however, that the diversity generated through that method is limited due to the use of homologous gene sequences (as defined in WO 95/22625).

Another disadvantage in the method of WO 95/22625 lies in the production of the random fragments by the cleavage of the template double-stranded polynucleotide.

A further reference of interest is WO 95/17413 describing a method of gene or DNA shuffling by recombination of DNA sequences either by recombination of synthesized double-stranded fragments or recombination of PCR generated sequences. According to the method described in WO 95/17413 the recombination has to be performed among DNA sequences with sufficient sequence homology to enable hybridization of the different sequences to be recombined.

WO 95/17413 therefore also entails the disadvantage that the diversity generated is relatively limited.

The present invention does not contain any steps involving production of random fragments by the cleavage of the template double-stranded polynucleotide, as described in WO 95/22625.

Further, WO 95/22625 relates to shuffling of homologous genes by random physical fragmentation and PCR-based reassembly, while the present invention relates to shuffling of heterologous genes without any physical fragmentation.

### SUMMARY OF THE INVENTION:

The problem to be solved by the present invention is to avoid the limitation of shuffling only homologous DNA sequences by providing a method to shuffle/recombine heterologous sequences of interest.

The solution is to use at least one "conserved sequence region", wherein there is a sufficient degree of homology between the heterologous sequences to be shuffled, as a "linking point" between said heterologous sequences.

Accordingly, a first aspect of the invention relates to a A method for shuffling heterologous DNA sequences encoding an enzymatic activity or activities of interest, the method comprising the following steps,
i) identifying one or more conserved region(s) in two or more of the heterologous sequences;
ii) constructing at least two sets of PCR primers directed to the conserved region(s), wherein the sequence regions between each set of PCR primers, including the primer sequences, have a homologous sequence overlap of at least 10 base pairs within the conserved region(s);
iii) performing two PCR amplification reactions with the at least two sets of PCR primers using the heterologous DNA sequences as templates;
iv) isolating and pooling PCR fragments generated in step iii) and performing a Sequence overlap extension PCR reaction (SOE-PCR) using said PCR fragments as templates; and
v) isolating PCR fragment(s) generated by the SOE-PCR reaction in step iv), wherein said isolated PCR fragment(s) comprise shuffled sequences and the partial DNA sequences originating from the homologous sequence overlaps in step ii) have at least 80% identity to one or more partial sequences in one or more of the original heterologous DNA sequences in step i).

The term "conserved region" denotes a sequence region (preferably of at least 10 bp), wherein there is a relatively high sequence identity between said heterologous sequences.

In order for the conserved region to be used as "linking point" between said heterologous sequences, the sequence identity between the heterologous sequences, within said conserved regions, is sufficiently high to enable hybridization of the heterologous sequences using said conserved region as hybridization point ("linking point").

### BRIEF DECRIPTION OF DRAWINGS

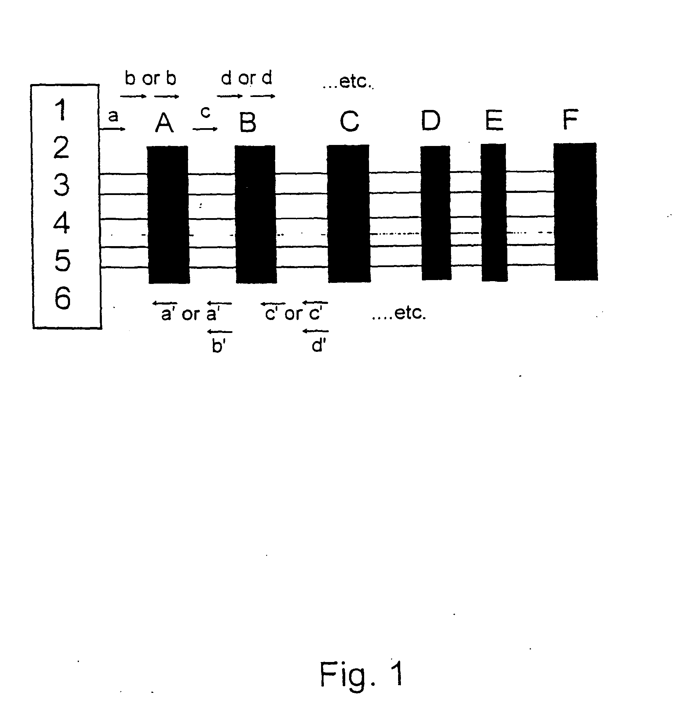

Fig. 1: Fig 1 illustrates the general concept of the invention, where six (1-6) sequences are aligned, wherein:
   i) the black boxes define mutual, common, conserved regions of the sequences of interest, and
   ii) the PCR primers named "a,a',b,b',etc.." are primers directed to the conserved regions. Primers ("a'" and "b"), ("b"' and "c") etc.. have a sequence overlap of preferably at least 10 bp, and
   iii) primers "z" and "z'" are primers directed to the flanking parts of the sequence area of the sequences of interest which are shuffled according to the method of the invention.

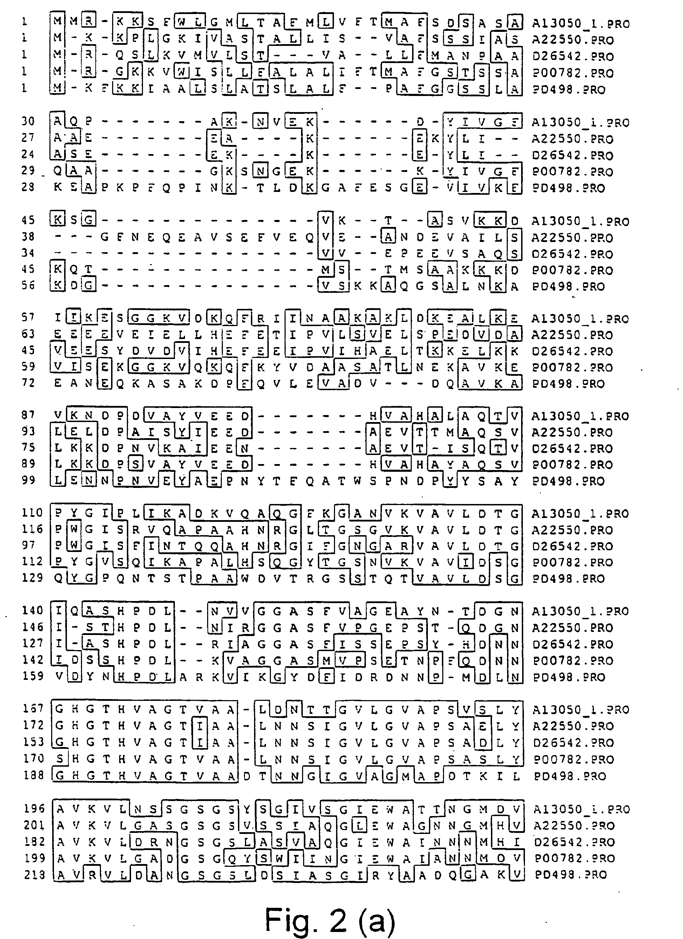

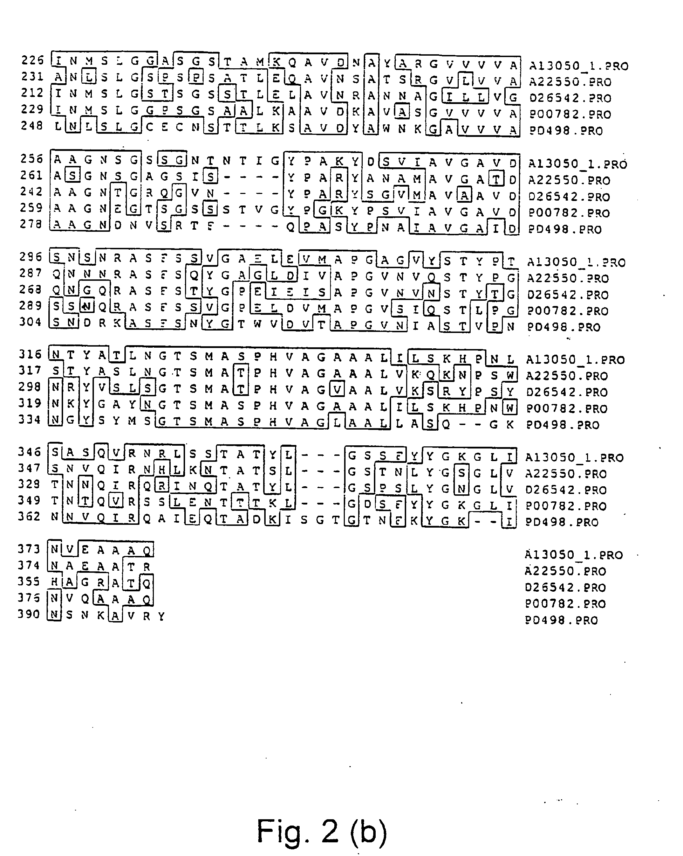

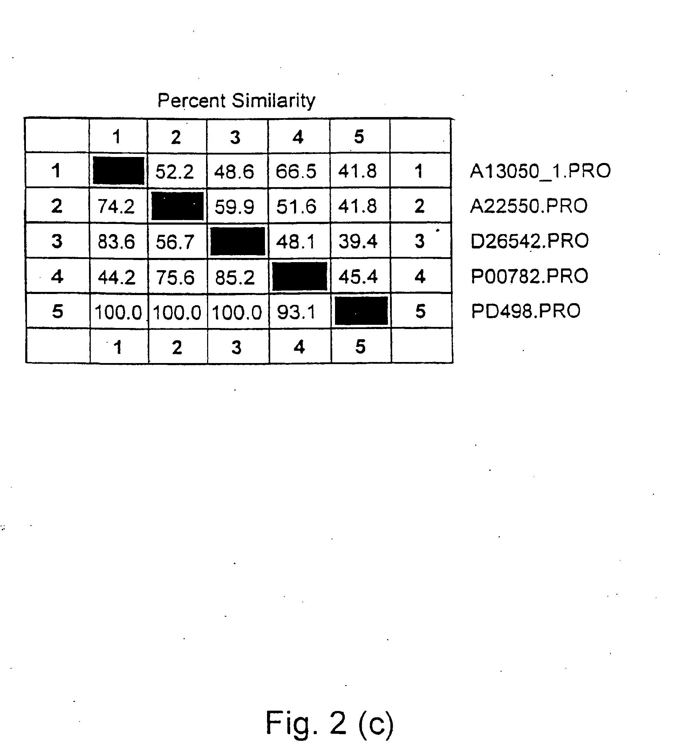

Fig 2: Shows an alignment of 5 protease (subtilase) amino acid sequences. Herein are a number of conserved regions that are boxed.

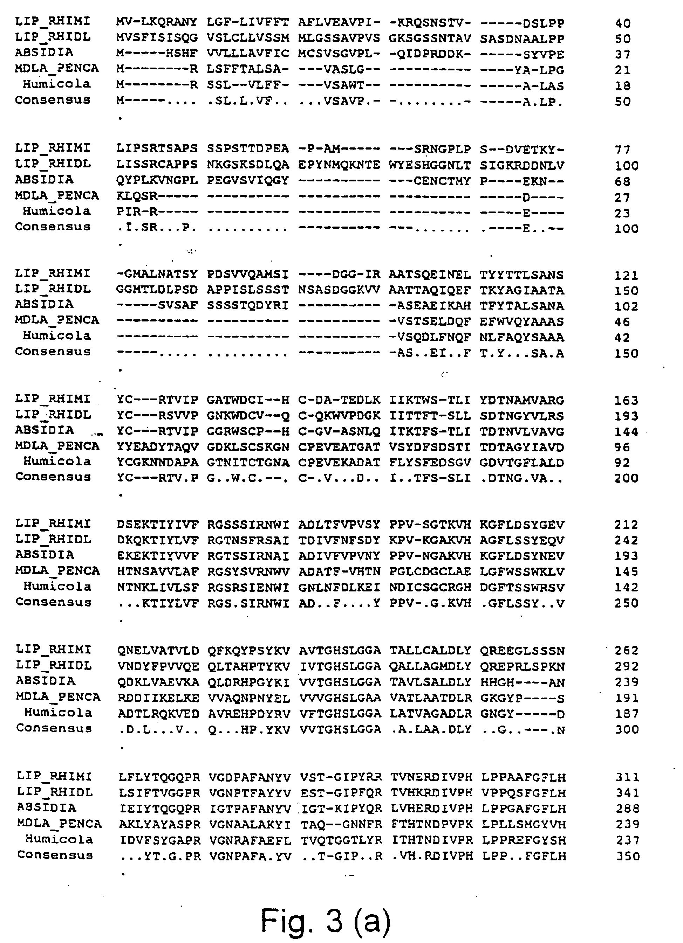

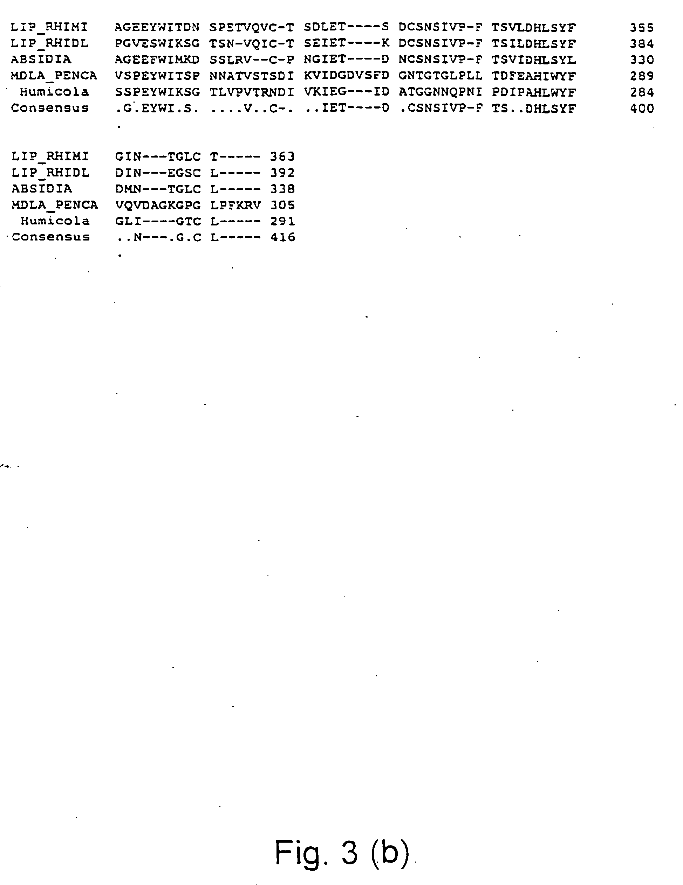

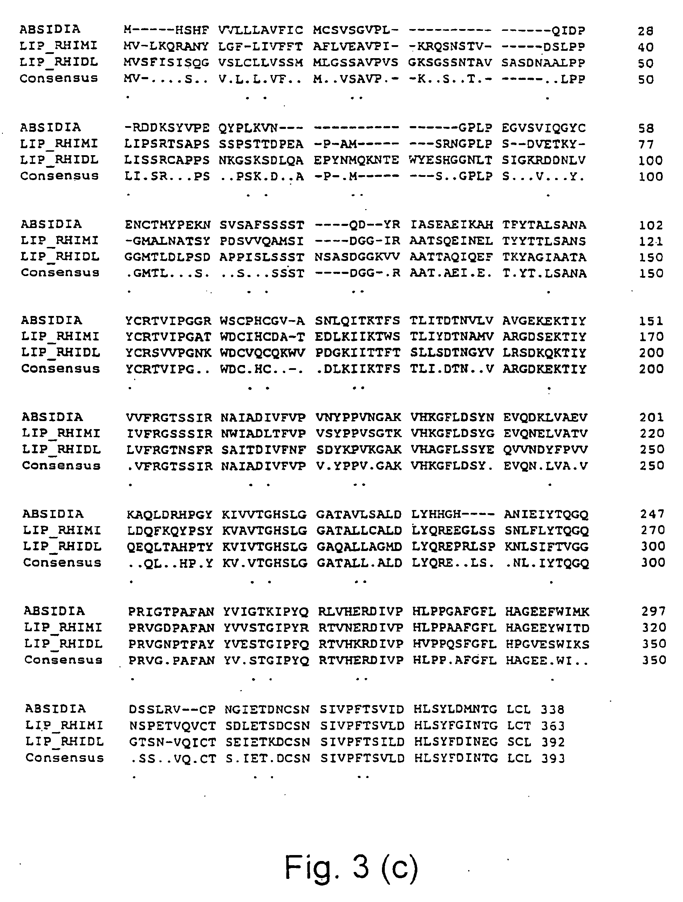

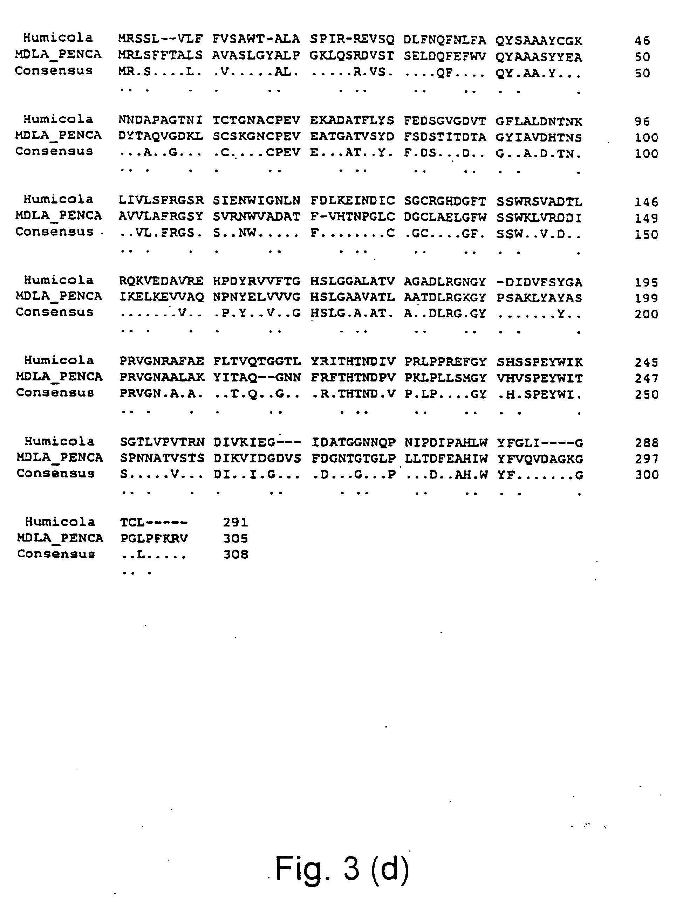

Fig 3: Shows an alignment of different lipases.

### DEFINITIONS

Prior to discussing this invention in further detail, the following terms will be defined.

"Shuffling": The term "shuffling" means recombination of nucleotide sequence(s) between two or more DNA sequences of interest resulting in output DNA sequences (i.e. DNA sequences having been subjected to a shuffling cycle) having a number of nucleotides exchanged, in comparison to the input DNA sequences (i.e. starting point DNA sequences of interest).

Alternatively, the term "shuffling" may be termed "recombining".

"Homology of DNA sequences": In the present context.the degree of DNA sequence homology is determined as the degree of identity between two sequences indicating a derivation of the first sequence from the second. The homology may suitably be determined by means of computer programs known in the art, such as GAP provided in the GCG program package (Program Manual for the Wisconsin Package, Version 8, August 1994, Genetics Computer Group, 575 Science Drive, Madison, Wisconsin, USA 53711)(Needleman, S.B. and Wunsch, C.D., (1970), Journal of Molecular Biology, 48, 443-453).

"Homologous": The term "homologous" means that one single-stranded nucleic acid sequence may hybridize to a complementary single-stranded nucleic acid sequence. The degree of hybridization may depend on a number of factors including the amount of identity between the sequences and the hybridization conditions such as temperature and salt concentration as discussed later (*vide infra*).

Using the computer program GAP (*vide supra*) with the following settings for DNA sequence comparison: GAP creation penalty of 5.0 and GAP extension penalty of 0.3, it is in the present context believed that two DNA sequences will be able to hybridize (using medium stringency hybridization conditions as defined below) if they mutually exhibit a degree of identity of at least 50%, more preferably at least 60%, more preferably at least 70%, more preferably at least 80%, more preferably at least 85%, and even more preferably at least 90%.

"Heterologous": Two DNA sequences are said to be heterologous if one of them comprises a partial sequence of at least 40 bp which does not exhibit a degree of identity of more than 50%, more preferably of more than 70%, more preferably of more than 80%, more preferably of more than 85%, more preferably of more than 90%, and even more preferably of more than 95%, of any partial sequence in the other. More preferably the first partial sequence is at least 60 bp, more preferably the first partial sequence is at least 80 bp, even more preferably the first partial sequence is at least 120 bp, and most preferably the first partial sequence is at least 500 bp.

"Hybridization:" Suitable experimental conditions for determining if two or more DNA sequences of interest do hybridize or not are herein defined as hybridization at medium stringency as described in detail below.

A suitable experimental low stringency hybridization protocol between two DNA sequences of interest involves presoaking of a filter containing the DNA fragments to hybridize in 5 x SSC (Sodium chloride/Sodium citrate, Sambrook et al. 1989) for 10 min, and prehybridization of the filter in a solution of 5 x SSC, 5 x Denhardt's solution (Sambrook et al. 1989), 0.5 % SDS and 100 µg/ml of denatured sonicated salmon sperm DNA (Sambrook et al. 1989), followed by hybridization in the same solution containing a concentration of 10ng/ml of a random-primed (Feinberg, A. P. and Vogelstein, B. (1983) Anal. Biochem. 132:6-13), 32P-dCTP-labeled (specific activity > 1 x 109 cpm/µg) probe (DNA sequence) for 12 hours at approx. 45°C. The filter is then washed twice for 30 minutes in 2 x SSC, 0.5 % SDS at least 55°C, more preferably at least 60°C, and even more preferably at least 65°C (high stringency).

Molecules to which the oligonucleotide probe hybridizes under these conditions are detected using an X-ray film.

"Alignment": The term "alignment" used herein in connection with an alignment of a number of DNA and/or amino acid sequences means that the sequences of interest are aligned in order to identify mutual/common sequences of homology/identity between the sequences of interest. This procedure is used to identify common "conserved regions" (*vide infra*), between sequences of interest. An alignment may suitably be determined by means of computer programs known in the art, such as PILEUP provided in the GCG program package (Program Manual for the Wisconsin Package, Version 8, August 1994, Genetics Computer Group, 575 Science Drive, Madison, Wisconsin, USA 53711)(Needleman, S.B. and Wunsch, C.D., (1970), Journal of Molecular Biology, 48, 443-453).

"Conserved regions:" The term "conserved region" used herein in connection with a "conserved region" between DNA and/or amino acid sequences of interest means a mutual, common sequence region of two or more sequences of interest, wherein there is a relatively high degree of sequence identity between two or more of the heterologous sequences of interest. In the present context a conserved region is preferably at least 10 base pairs (bp), more preferably at least 20 bp, and even more preferably at least 30 bp.

Using the computer program GAP (*vide supra*) with the following settings for DNA sequence comparison: GAP creation penalty of 5.0 and GAP extension penalty of 0.3, the degree of DNA sequence identity within the conserved region, between two or more of the heterologous sequences of interest, is preferably at least 80%, more preferably at least 85%, more preferably at least 90%, and even more preferably at least 95%.

"Primer": The term "primer" used herein, especially in connection with a PCR reaction, is a primer (especially a "PCR-primer") defined and constructed according to general standard specification known in the art ("PCR A practical approach" IRL Press, (1991)).

"A primer directed to a sequence:" The term "a primer directed to a sequence" means that the primer (preferably to be used in a PCR reaction) is constructed so as to exhibit at least 80% degree of sequence identity to the sequence part of interest, more preferably at least 90% degree of sequence identity to the sequence part of interest; which said primer consequently is "directed to".

"Sequence overlap extension PCR reaction (SOE-PCR)": The term "SOE-PCR" is a standard PCR reaction protocol known in the art, and in the present context it is defined and performed according to standard protocols defined in the art ("PCR A practical approach" IRL Press, (1991)).

"Flanking": The term "flanking" used herein in connection with DNA sequences comprised in a PCR-fragment means the outmost end partial sequences of the PCR-fragment, both in the 5' and 3' ends of the PCR fragment.

"Subtilases": A serine protease is an enzyme which catalyzes the hydrolysis of peptide bonds, and in which there is an essential serine residue at the active site (White, Handler and Smith, 1973 "Principles of Biochemistry," Fifth Edition, McGraw-Hill Book Company, NY, pp. 271-272).

The bacterial serine proteases have molecular weights in the range of 20,000 to 45,000 Daltons. They are inhibited by diisopropylfluorophosphate. They hydrolyze simple terminal esters and are similar in activity to eukaryotic chymotrypsin, also a serine protease. A more narrow term, alkaline protease, covering a sub-group, reflects the high pH optimum of some of the serine proteases, from pH 9.0 to 11.0 (for review, see Priest (1977) Bacteriological Rev. 41 711-753).

A sub-group of the serine proteases tentatively designated subtilases has been proposed by Siezen et al., Protein Engng. 4 (1991) 719-737. They are defined by homology analysis of more than 40 amino acid sequences of serine proteases previously referred to as subtilisin-like proteases.

### DETAILED DESCRIPTION OF THE INVENTION

### A method for shuffling heterologous sequences of interest

In a preferred embodiment the fragments generated in step iii) of the first aspect of the invention is generated by use of PCR technology.

Accordingly, an aspect of the invention relates to a method of shuffling of heterologous DNA sequences of interest, according to the first aspect of the invention, comprising the following steps
i) identification of one or more conserved region(s) (hereafter named "A,B,C" etc..) in two or more of the heterologous sequences;
ii) construction of at least two sets of PCR primers (each set comprising a sense and an anti-sense primer) for one or more conserved region(s) identified in i) wherein
   in one set the sense primer (named: "a"=sense primer) is directed to a sequence region 5' (sense strand) of said conserved region (*e.g*. conserved region "A"), and the anti-sense primer (named "a'"=anti-sense primer) is directed either to a sequence region 3' (sense strand) of said conserved region or directed to a sequence region at least partially within said conserved region,
   and in another set the sense primers (named: "b"=sense primer) is; directed either to a sequence region 5' (sense, strand) of said conserved region or directed to a sequences region at least partially within said conserved region and the anti-sense primer (named: "b'"=anti-sense primer) is directed to a sequence region 3' (sense strand) of said conserved region (*e.g*. conserved region "A"), and
   the two sequence regions defined by the regions between primer set "a" and "a'" and "b" and "b'" (both said regions is including the actual primer sequences) have a homologous sequence overlap of at least 10 base pairs (bp) within the conserved region;
iii) for one or more identified conserved regions of interest in step i) two PCR amplification reactions are performed with the heterologous DNA sequences in step i) as template, and where
   one of the PCR reactions uses the 5' primer set identified in step ii) (e.g. named "a","a'") and the second PCR reaction uses the 3' primer set identified in step ii) (e.g. named "b","b'");
iv) isolating and pooling PCR fragments generated in step iii) and performing a Sequence overlap extension PCR reaction (SOE-PCR) using said isolated PCR fragments as templates; and
v) isolation of the PCR fragment obtained in step (v), wherein said isolated PCR fragment comprises numerous different shuffled sequences containing a shuffled mixture of the PCR fragments, wherein said shuffled sequences are
   characterized in that the partial DNA sequences, originating from the homologous sequence overlaps in step ii), have at least 80% identity to one or more partial sequences in one or more of the original heterologous DNA sequences in step i).

An embodiment of the invention relates to a method of producing a shuffled protein having a desired biological activity comprising in addition to the steps i) to vi) immediately above the further steps:
vi) expressing the numerous different recombinant proteins encoded by the numerous different shuffled sequences in step vi); and
vii) screen or select the numerous different recombinant proteins from step vii) in a suitable screening or selection system for one or more recombinant protein(s) having a desired activity.

### Heterologous DNA sequences

The method of the present invention may be used to shuffle basically all heterologous DNA sequences of interest.

Preferably, it is used to shuffle heterologous DNA sequences encoding an enzymatic activity, such as amylase, lipase, cutinase, cellulase, oxidase, phytase, and protease activity.

An further advantage of the present method is that it makes it possible to shuffle heterologous sequences encoding different activities, *e.g*. different enzymatic activities.

The method of the invention is in particular suitable to shuffle heterologous DNA sequences encoding a protease activity, in particular a subtilase activity.

A number of subtilase DNA sequences are published in the art. A number of those subtilase DNA sequences are in the present context heterologous DNA sequences, and it is generally believed that they are mutually too heterologous to be shuffled by the shuffling methods presently known in the art (WO 95/17413, WO 95/22625). However the method according to the invention enables shuffling of such sequences. For further details reference is made to a working example herein (*vide infra*).

Further, the present invention is suitable to shuffle different lipase sequences. For further details reference is made to a working example herein (*vide infra*).

The heterologous DNA sequences used as templates may beforehand have been cloned into suitable vectors, such as a plasmid. Alternatively, a PCR-reaction may be performed directly on microorganisms known to comprise the DNA sequence of interest according to standard PCR protocols known in the art.

### Identification of one or more conserved regions in heterologous sequences:

Identification of conserved regions may be done by an alignment of the heterologous sequences by standard computer programs (*vide supra*).

Alternatively, the method may be performed on completely new sequences, where the relevant "conserved regions" are chosen as conserved regions which are known in the art to be conserved regions for this particular class of proteins.

*E.g.*, the method may be used to shuffle completely unknown subtilase sequences, which are known to be very conserved in *e.g*. regions around the active site amino acids. PCR reaction may then be performed directly on new unknown strains with primers directed to those conserved regions.

### PCR-primers

The PCR primers are constructed according to the standard descriptions in the art. Preferably, they are 10-75 base pairs (bp) long.

### Homologous sequence overlap

In step ii) of claim 3 of the invention the two sequence regions defined by the regions between primer set "a" and "a'" and "b" and "b'" (both said regions is including the actual primer sequences) have a homologous sequence overlap of at least 10 base pairs (bp) within the conserved region.

Said homologous sequence overlap is more preferably of at least 15 bp, more preferably of at least 20 bp, and even more preferably of at least 35 bp.

The homologous sequence overlaps in step ii) of claim 3 have at least 80% identity to one or more partial sequences in one or more of the original heterologous DNA sequences in step i) of said claim, more preferably the homologous sequence overlaps in step ii) have at least 90% identity to one or more partial sequences in one or more of the original heterologous DNA sequences in step i) of said claim, and even more preferably the homologous sequence overlaps in step ii) have at least 95% identity to one or more partial sequences in one or more of the original heterologous DNA sequences in step i) of said claim.

### PCR-reactions

If not otherwise mentioned the PCR-reaction performed according to the invention is performed according to standard protocols known in the art.

The term "Isolation of PCR fragment" is intended to cover an aliquot containing the PCR fragment. However, the PCR fragment is preferably isolated to an extent which removes surplus of primers, nucleotides, etc.

Further, the fragment used for SOE-PCR in step v) of claim 3, may alternatively be generated by other processes than the PCR amplification process described in step iii) of said claim. Suitable fragments used for the SOE-PCR in step v), may *e.g*. be generated by cutting out suitable fragments by restriction enzyme digestion at appropriate sites (e.g. restriction sites situated on each site of a conserved region identified in step i). Such alternative processes for generating such suitable fragments for use in the SOE-PCR in step v) are considered within the scope of the invention.

In an embodiment of the invention the PCR DNA fragment(s) is(are) prepared under conditions resulting in a low, medium or high random mutagenesis frequency.

To obtain low mutagenesis frequency the DNA sequence(s) (comprising the DNA fragment(s)) may be prepared by a standard PCR amplification method (US 4,683,202 or Saiki et al., (1988), Science 239, 487 - 491).

A medium or high mutagenesis frequency may be obtained by performing the PCR amplification under conditions which increase the misincorporation of nucleotides, for instance as described by Deshler, (1992), GATA 9(4), 103-106; Leung et al., (1989), Technique, Vol. 1, No. 1, 11-15.

### Final shuffles sequences

One of the advantages of the present invention is that the final "shuffled sequences" in step vi) of claim 3 of the present invention only comprise sequence information which is originally derived from the original heterologous sequences of interest in step i) of said claim. The present invention does not use artificially made "linker sequences" to recombine one or more of the heterologous sequences, which is a strategy known in the art to e.g. be able to shuffle different domains in proteins, wherein each domain is encoded by different heterologous sequences (WO 95/17413).

Accordingly, the invention relates to a method characterized in that each of the shuffled sequences, the partial DNA sequences, originating from the homologous sequence overlaps in step ii), only contains sequence information which is originally derived from the original heterologous sequences in step i) (in the first to third aspect of the invention) (i.e. said "homologous sequence overlaps" in step ii) has at least 80% identity to one or more partial sequences in one or more of the original heterologous DNA sequences in step i).

More preferably, the "homologous sequence overlaps" in step ii) have at least 90% identity to one or more partial sequences in one or more of the original heterologous DNA sequences in step i); and even more preferably the "homologous sequence overlaps" in step ii) have at least 95% identity to one or more partial sequences in one or more of the original heterologous DNA sequences in step i), and most preferably the "homologous sequence overlaps" in step ii) have 100% identity to one or more partial sequences in one or more of the original heterologous DNA sequences in step i).

### Expressing the recombinant protein from the shuffled sequences

Expression of the recombinant protein encoded by the shuffled sequence of the present invention may be performed by use of standard expression vectors and corresponding expression systems known in the art.

### Suitable screening or selection system

In its second aspect the present invention relates to a method for producing one or more recombinant protein(s) having a desired biological activity.

A suitable screening or selection system will depend on the desired biological activity.

A number of suitable screening or selection systems to screen or select for a desired biological activity are described in the art. Examples are:
Strauberg et al. (Biotechnology 13: 669-673 (1995), which describes a screening system to screen for subtilisin variants having a calcium-independent stability;
Bryan et al. (Proteins 1:326-334 (1986)), which describes a screening assay to screen for proteases having enhanced thermal stability; and
WO 97/04079 which describes a screening assay to screen for lipases having an improved wash performance in washing detergents.

A preferred embodiment of the invention comprises screening or selection of recombinant protein(s), wherein the desired biological activity is performance in dish-washing or laundry detergents. Examples of suitable dish-washing or laundry detergents are disclosed in WO 97/04079 and WO 95/30011.

The invention is described in further detail in the following examples which are not in any way intended to limit the scope of the invention.

### MATERIALS AND METHODS

### Strains

### E. coli strain: DH10B (Life Technologies)

Bacillus subtilis strain: DN1885 amyE. A derivative of B,s 168RUB200 (J. Bacteriology 172:4315-4321 (1990))

### Plasmids

pKH400: pKH400 was constructed from pJS3 (E. coli - B. subtilis shuttle vector containing a synthetic gene encoding for subtilase 309 (described by Jacob Schiødt et al. in Protein and Peptide letters 3:39-44 (1996)), by introduction of two BamHI sites at positions 1841 and 3992.

### Protease sequences used for shuffling

GenBank entries A13050_1, D26542, A22550, Swiss-Prot entry SUBT_BACAM P00782, and PD498 (Patent Application No. WO 96/34963).

### General molecular biology methods

Unless otherwise mentioned the DNA manipulations and transformations were performed using standard methods of molecular biology (Sambrook et al. (1989) Molecular cloning: A laboratory manual, Cold Spring Harbor lab., Cold Spring Harbor, NY; Ausubel, F. M. et al. (eds.) "Current protocols in Molecular Biology". John Wiley and Sons, 1995; Harwood, C. R., and Cutting, S. M. (eds.) "Molecular Biological Methods for Bacillus". John Wiley and Sons, 1990).

Enzymes for DNA manipulations were used according to the specifications of the suppliers.

### Enzymes for DNA manipulations

Unless otherwise mentioned all enzymes for DNA manipulation, such as *e.g*. restiction endonucleases, ligases etc., are obtained from New England Biolabs, Inc.

### EXAMPLES

### EXAMPLE 1

### A) Vector construction

### 1) Amplification of the pre-pro sequences

Host cells harboring the plasmid DNA encoding the full length enzymes A13050_1 (GenBank), SUBT_BACAM P00782 (Swiss-Prot), D26542 (GenBank), A22550 (GenBank), and PD498 (Patent Application No. WO 96/34963) were starting material. By standard mini-prep isolation of plasmid DNA, purified DNA was obtained. With these template DNAS, 5 standard PCRs were performed to amplify the respective pre-pro sequences. The fragments were generated using the proof reading Pwo DNA polymerase (Boehringer Mannheim) and the following sets of primers directed against the very N- and C-termini of the respective pre-pro sequences:
A13050_1
   TiK111: 5' GAG GAG GGA AAC CGA ATG AGG AAA AAG AGT TTT TGG.
   TiK117: 5' CGC GGT CGG GTA CCG TTT GCG CCA AGG CAT G.
SUBT_BACAM P00782
   T1K112: 5' GAG GAG GGA AAC CGA ATG AGA GGC AAA AAA GTA TGG.
   TiK118: 5' CGC GGT CGG GTA CCG ACT GCG CGT ACG CAT G.
D26542
   TiK110: 5' GAG GAG GGA AAC CGA ATG AGA CAA AGT CTA AAA GTT ATG.
   TiK116: 5' CGC GGT CGG GTA CCG TTT GAC TGA TGG TTA CTT C.
A22550
   TiK109: 5' GAG GAG GGA AAC CGA ATG AAG AAA CCG TTG GGG.
   TiK115: 5' CGC GGT CGG GTA CCG ATT GCG CCA TTG TCG TTA C.
PD498
   TiK113: 5' GAG GAG GGA AAC CGA ATG AAG TTC AAA AAA ATA GCC.
   TiK119: 5' CGC GGT CGG GTA CCG CAG AAT AGT AAG GGT CAT TC.

The obtained DNA fragments of a length between 300-400 bp were purified by agarose gel-electrophoresis with subsequent gel extraction (QIAGEN) and subjected to assembly by splice-by-overlap extension PCR (SOE-PCR).

### 2) SOE-PCR

The pre-pro fragments were then separately spliced by SOE-PCR to the 3' part of the promoter of the vector pKH400. The 3' part of the promoter was obtained by standard PCR with the Pwo DNA polymerase using 1 ng of pKH400 as template and the primers:
TiK106: 5' CGA CGG CCA GCA TTG G.
TiK107: 5' CAT TCG GTT TCC CTC CTC.
The resulting 160 bp fragment was gel-purified. Subsequently, 5 SOE-PCRs were performed under standard conditions (Pwo DNA polymerase) using as template each of the 5 pre-pro sequences mixed with equal molar amounts of the 3' part of the promoter. The assembling primers were:
TiK120: 5' CTT TGA TAC GTT TAA ACT ACC.
TiK121: 5' CGC GGT CGG GTA CCG.
The obtained fragments were also gel-purified.

### 3) Insertion of the pre-pro sequences into the pKH400 shuttle vector

The pKH400 vector was cut with Pme I and Acc65 I to remove the existing linker sequence. The 5 purified SOE-PCR fragments from 2) were also digested with the same enzymes and gel-purified. Only with the SOE-PCR of the SUBT_BACAM P00782 pre-pro sequence special caution was required because it contains an internal Pme I-site so that a partial digest was performed. In separate standard ligation mixes the pre-pro fragments were then ligated to the pKH400 vector. After transformation of DH10B E.coli cells, colonies were selected on ampicillin containing media. Correctly transformed cells were identified by control digest and sequenced. The thus obtained vectors were named pTK4001-4005.

B)Preparation of the small fragments of the proteases A13050_1 (GenBank), SUBT_BACAM P00782 (Swiss-Prot), D26542 (GenBank), A22550 (GenBank), and PD498 (Patent Application No. WO 96/34963).

I) Standard PCR reactions were assembled with 0.5 µl of mini-prep DNA of each protease gene as templates. Since these five protease genes shall be fragmented into six fragments (I-VI), 30 PCRs are required (see fig 1). The Ampli-Taq polymerase (5U) was used in combination with the following primer sets (the numbering corresponds to the amino acid position in A22550). If there are primers labeled #.1, #.2, etc., then equal molar amounts of them are mixed prior to PCR and treated as one primer in the PCR:
Set I)
   TiK122.1 (116-124).
   5' CCG GCG CAG GCG GTA CCX TRS GGX ATW XCX CXX RTX MAA GC.
   TiK122.2 (116-124)
   5' CCG GCG CAG GCG GTA CCX TRS GGX ATW XCA WWC ATX WAT AC.
   TiK123 (174-180)
   5' GTT CCX GCX ACR TGX GTX CC.
Set II)
   TiK124 (174-180)
   5' GGX ACX CAY GTX GCX GGA AC.
   TiK125.1 (217-223)
   5' GCC CAC TSX AKX CCG YTX AC.
   TiK125.2 (217-223)
   5' GCC CAC TSX AKX CCT YGX GC.
   TiK125.3 (217-223)
   5' GCC CAX TSR AKX CCK XXX RCW AT.
Set III)
   TiK126.1 (217-223)
   5' GTX ARC GGX MTX SAG TGG GC.
   TiK126.2 (217-223)
   5' GCX CRA GGX MTX SAG TGG GC.
   TiK126.3 (217-223)
   5' TWG CYC AAG GWW TXS AXT GKR.
   TiK126.5 (217-223)
   5' TWG CTC AAG GHH THS ART GG.
   TiK127.1 (255-261)
   5' GCX GCX ACX ACX ASX ACX CC.
   TiK1272 (255-261)
   5' GCY SCW AYW AMX AGW AYA YCA.
Set IV)
   TiK128.1 (255-261)
   5' GGX GTX STX GTX GTX GCX GC.
   TiK128.2 (255-261)
   5' TGR TRT WCT MKT WRT WGS RGC.
   TiK129.1 (292-299)
   5' GBX CCX ACR YTX GAR AAW GAX G.
   TiK129.2 (292-299)
   5' GBX CCR TAC TGX GAR AAR CTX G.
   TiK129.3 (292-299)
   5' GKX CCA TAC KKA GAR AAR YTT G.
   TiK129.5 (292-299)
   5' GKR CCA TAC KKA GAR AAG YTT G.
Set V)
   TiK130.1 (292-299)
   5' CXT CWT TYT CXA RYG TXG GXV C.
   TiK130.2 (292-299)
   5' CXA GYT TYT CXC AGT AYG GXV C.
   TiK130.3 (292-299)
   5' CAA GYT TCT CTM MGT ATG GSM C.
   TiK130.5 (292-299)
   5' CAA GTT TCT CTC AGT ATG GGA C.
   TiK131.1 (324-330)
   5' GGX GWX GCC ATX GAY GTX CC.
   TiK131.2 (324-330)
   5' GGA GTA GCC ATX GAX GTW CC.
Set VI)
   TiK132.1 (324-330)
   5' GGX ACR TCX ATG GCX WCX CC.
   TiK132.2 (324-330)
   5' GGW ACX TCX ATG GCA WCX CC.
   TiK133.1 (375-380)
   5' CGG CCC CGA CGC GTT TAC YGX RYX GCX SYY TSX RC.
   TiK133.2 (375-380)
   5' CGG CCC CGA CGC GTT TAT CKT RYX GCX XXY TYW G.
   TiK133.3(375-380)
   5' CGG CCC CGA CGC GTT TAT CKT RCX GCX GCX TYT GMR TT.
   TiK133.4 (375-380)
   5' CGG CCC CGA CGC GTT TAT CTT ACG GCA GCC TCA GC.
   (X = deoxy-inosine, Y = 50% C + 50% T, R = 50% A + 50% G, S = 50%
   C + 50% G, W = 50% A + 50 % T, K = 50 % T + 50 % G, M = 50 % A + 50%
   C, B = 33.3% C + 33.3% G + 33.3% T, V = 33.3% C + 33.3% G + 33.3% A, H = 33.3% C + 33.3% A + 33.3%).

After 30 cycles at annealing temperatures ranging from 40-60°C the amplified fragments were gel-purified and recovered.

### 2) SOE-PCR to randomly assemble the small fragments

Equimolar amounts of each of the purified fragments were taken and mixed in one tube as templates for assembly in an otherwise standard SOE-PCR with Ampli-Taq polymerase. The external primers used are:

| | |
|---|---|
| TiK134.1: | CCG GCG CAG GCG GTA CC. |
| TiK135.1: | CGG CCC CGA CGC GTT TA. |

Also the primer pairs

| | |
|---|---|
| TiK134.2: | GGC GCA GGC GGT AC. |
| TiK135.2: | GCC CCG ACG CGT TTA. |

and

| | |
|---|---|
| TiK134.3: | CGC AGG CGG TAC. |
| TiK135.3: | CCC GAC GCG TT. |

can be used. The annealing temperatures are ranging from 40°C to 70°C.

The re-assembly is also achieved by sequentially reassembling all conceivable combinations of fragments, e.g.: In tube 1 all seven fragments obtained by PCR with the primers of set I (see above, B1-2) are mixed, in tube 2 fragments obtained by PCR with the primers of set II are mixed, in tube 3 fragments obtained by PCR with the primers of set III are mixed, in tube 4 fragments obtained by PCR with the primers of set IV are mixed, in tube 5 fragments obtained by PCR with the primers of set V are mixed, in tube 6 fragments obtained by PCR with the primers of set VI are mixed.

Then, a SOE-PCR is performed by mixing aliquots of tube 1 and 2 and using the resulting mixture as template for a primary SOE-PCR with corresponding external primers. The same is performed with mixtures of aliquots of tubes 3 and 4 as well as tubes 5 and 6. The respective external primer pairs are TiK134.#/125.# for fragments 1 and 2, TiK126.#/129.# for fragments 3 and 4, and TiK 130.#/135.# for fragments 5 and 6. The amplified assembled fragments of about 340, 260, and 280 bp length, respectively, are purified by agarose gel electrophoresis. In a secondary SOE-PCR the obtained fragments are mixed and assembled using primer pair TiK134.#/135.# as external primers. The obtained full-length protease genes are gel-purified as described above.

In another example, aliquots of tubes 1, 2, and 3 are mixed and re-assembled by a primary SOE-PCR with primer pair TiK134.#/127.#. Aliquots of tubes 4, 5, and 6 are also mixed in another tube and re-assembled by another SOE-PCR using the primers TiK128.#/135.#. The generated fragments of about 450 bp length are purified as described above, mixed and reassembled in a secondary SOE-PCR with external primers TiK134.#/135.#. The obtained full-length protease genes are gel-purified as described above.

In principle, every combination of fragments may be assembled in separate SOE-PCRs. In subsequent SOE-PCRs the obtained assembled units are assembled to larger units until the final full length gene is obtained. The overall number of SOE-PCRs used for that purpose is only limited by experimental capacity. The only prerequisite which is inherent to SOE-PCR is that the fragments to be assembled must contain a sequence overlap as defined earlier.

### C) Cloning of the SOE-PCR-derived full-length protease-hybrids to yield library #1

The full-length protease-hybrid genes from step B2) as well as the newly constructed shuttle vectors pTK4001-4005 from A3) are separately digested with Acc65 I and Mlu I. In standard ligation procedures the protease genes are separately ligated to each of the five vectors pTK4001-4005 and transformed into E.coli DH10B. Selection of correctly transformed cells is performed with ampicillin. DNA of these clones is prepared and designated library #1. The library size is about 10⁶ independent transformants.

### D) Screening of library #1

Aliquots of library #1 are used to transform Bacilli cells DN1885. The transformants are screened for the desired properties.

By this method and using a standard protease activity assay to screen for the desired property in step D) above a number of new shuffled subtilisins with a desired property were identified.

The results are indicated in Table 1 below.

**Table 1**

| Clone | pre-pro | frag.1 (5') | frag.2 | frag.3 | frag.4 | frag.5 | frag.6 (3') |
|---|---|---|---|---|---|---|---|
| 8 | BPN | Sav | Sav | Sav | Sav | Sav | Sav |
| 6 | Alc | Sav | Sav | Sav | Sav | Sav | Sav |
| 12 | Esp | Sav | Sav | Sav | Sav | Sav | Sav |
| 10 | PD498 | Sav | Sav | Sav | Sav | Sav | Sav |
| 4 | Esp | PD138 | Esp | Esp | Esp | Esp | JA16 |
| 22 | Alc | PD138 | Esp | Esp | Esp | Esp | JA16 |
| 11 | PD498 | PD138 | Esp | Esp | Esp | Esp | JA16 |
| 1 | Alc | PD138 | Esp | PD138 | Esp | Esp | JA16 |
| 3 | BPN | PD138 | Esp | Esp | PD138 | Sav | Sav |
| 17 | Esp | PD138 | PD138 | Esp | Esp | Esp | JA16 |
| 19 | PD498 | Alc | BPN | Esp | Esp | Esp | JA16 |
| 16 | Alc | Alc | BPN | Esp | PD138 | Esp | JA16 |

| | | | | | | | |
|---|---|---|---|---|---|---|---|
| Identity of clones: Alcalase: A13050_1 (GenBank) BPN': Poo782 (SwisProt) Esperase: D26542 (GenBank) Savinase: A22550 (GenBank) PD498: WO 96/34963 JA16: WO 92/17576 PD138 WO 93/18140 | | | | | | | |

23 clones having protease activity were identified of which 12 were different. Clones 8, 9, 18, 20, 23 were the same; clones 6, 15, 21 were the same, clones 12, 14 were the same, clones 10, 13 were the same, and clones 4, 7 were the same. In respect of mature enzymes 7 different were identified.

From Table 1 it is seen that the process of the invention makes it possible to obtain active proteins representing combinations of proteins quite distantly related.

### Example 2

The same methods as described in example 1 can be used for amplification of PCR fragments from fungal lipases.

The fungal lipases from the following fungi are aligned using the alignment program from Geneworks (using the following parameters:cost to open a gap = 5, cost to lengthen a gap = 25, Minimum Diagonal lLength = 4, Maximum Diagonal Length = 10, Consensus cutoff = 50%): Rhizomucor Miehei (LIP_RHIMI from the Swiss Prot data base), Rhizopus Delemar (LIP_RHIDL from the Swiss Prot data base), Penecillium camenbertii (MDLA PENCA from the Swiss Prot data base) Absidia reflexa (WO 96/13578) and Humicola lanuginosa (US 5536661).

Primers for amplification of Absidia (Absidia), Rhizopus (LIP_RHIDL) and Rhizomucor(LIP_RHIMI) lipase genes for shuffling *N: according to the IUPAC nomenclature means all 4 bases (A, T, G, C)*.
Set 1)

| | |
|---|---|
| 5' primer for YCRT/SVI/VPG: | TAY TGY MGR ACN GTN ATH CCN GG or TAY TGY MGR AGY/TCN GTN GTN CCN GG |
| 3' primer for VFRGT/S: | NSW NCC YCK RAA NAC |

Set 2)

| | |
|---|---|
| 5' primer for VFRGT/S: | GTN TTY MGR GGN WSN |
| 3' primer for KVHK/AGF: | RAA NCC YTT RTG NAC YTT or |
| | RAA NCC NGC RTG NAC YTT |

Set 3)

| | |
|---|---|
| 5' primer for KVHK/AGF: | AAR GTN CAY AAR GGN TTY or |
| | AAR GTN CAY GCN GGN TTY |
| 3' primer for VTGHSLGG: | CC NCC YAR NGA RTG NCC NGT NAC or |
| | CC NCC YAR RCT RTG NCC NGT NAC |

Set 4)

| | |
|---|---|
| 5' primer for VTGHSLGG: | GTN ACN GGN CAY TCN YTR GGN GG or |
| | GTN ACN GGN CAY AGY YTR GGN GG |
| 3' primer for FGFLH: RTG | YAR RAA NCC RAA |

Set 5)

| | |
|---|---|
| 5' primer for FGFLH: | TTY GGN TTY YTR CAY |
| 3' primer for IVPFT: | NGT RAA NGG NAC DAT |

Primers for amplification of Humicola lanuginosa(Humicola) and Penicillium camenbertii (MDLA_PENCA) lipase genes for shuffling
Set 1)

| | |
|---|---|
| 5' primer for CPEVE: | TGY CCN GAR GTN GAR |
| 3' primer for VLS/AFRG: | NCC YCK RAA NGM YAR NAC |

Set 2)

| | |
|---|---|
| 5' primer for VLS/AFRG: | GTN YTR KCN TTY MGR GGN |
| 3' primer for GFT/WSSW: | CCA NGA NGA NGT RAA NCC or |
| | CCA RSW RSW CCA RAA NCC |

Set 3)

| | |
|---|---|
| 5' primer for GFT/WSSW: | GGN TTY ACN TCN TCN TGG or |
| | GGN TTY TGG WSY WSY TGG |
| 3' primer for GHSLGG/AA: | NGC NSC NCC YAR NGA RTG NCC or |
| | NGC NSC NCC YAR RCT RTG NCC |

Set 4)

| | |
|---|---|
| 5' primer for GHSLGG/AA: | GGN CAY TCN YTR GGN GSN GCN or |
| | GGN CAY AGY YTR GGN GSN GCN |
| 3' primer for PRVGN: | RTT NCC NAC YCK NGG |

Set 5)

| | |
|---|---|
| 5' primer for PRVGN: | CCN MGR GTN GGN AAY |
| 3' primer for THTND: | RTC RTT NGT RTG NGT |

Set 6)

| | |
|---|---|
| 5' primer for THTND: | ACN CAY ACN AAY GAY |
| 3' primer for PEYWI: | DAT CCA RTA YTC NGG |

Set 7)

| | |
|---|---|
| 5' primer for PEYWI: | CCN GAR TAY TGG ATH |
| 3' primer for AHL/IWYF: | RAA RTA CCA DAK RTG NGC |

Primers for shuffling of all five genes:
Set 1)
   5' primer for AN/TA/SYCR: GCN AMY KCN TAY TGY MG for Absidia, Rhizopus and Rhizomucor sequences
   5' primer for AN/TA/SYCGKNNDA: GCN AMY KCN TAY TGY GGN AAR AAY AAY GAY GC for Humicola
   5' primer for AN/TA/SYCEADYTA: GCN AMY KCN TAY TGY GAR GCN GAY TAY ACN GC for P. camenbertii
   3' primer for E/OKTIY: RTA DAT NGT YTT YTS for Absidia, Rhizopus and Rhizomucor sequences
   3' primer for ALDNTE/OKTIY: RTA DAT NGT YTT YTS NGT RTT RTC YAR NGC for Humicola
   3' primer for AVDHTE/OKTIY : RTA DAT NGT YTT YTS NGT RTG RTC NAC NGC for P. camenbertii
Set 2)
   5' primer for E/OKTIY: SAR AAR ACN ATH TAY for Absidia, Rhizopus and Rhizomucor sequences
   5' primer for E/OKTIYLA/SFRG: SAR AAR ACN ATH TAY YTR KCN TTY MGR GGN for the two other sequences
   3' primer for KVHK/AGF: RAA NCC YTT RTG NAC YTT or RAA NCC NGC RTG NAC YTT for Absidia, Rhizopus and Rhizomucor sequences
   3' primer for ICSGCKVHK/AGF: RAA NCC YTT RTG NAC YTT RCA NCC NGA RCA DAT or RAA NCC NGC RTG NAC YTT RCA NCC NGA RCA DAT for Humicola
   3' primer for LCDGCKVHK/AGF: RAA NCC YTT RTG NAC YTT RCA NCC RTC RCA YAR or RAA NCC NGC RTG NAC YTT RCA NCC RTC RCA YAR for P. camenbertii
Set 3)
   5' primer for KVHK/AGF: AAR GTN CAY AAR GGN TTY or AAR GTN CAY GCN GGN TTY for Absidia, Rhizopus and Rhizomucor sequences
   5' primer for KVHK/AGFTSSW: AAR GTN CAY AAR GGN TTY ACN TCN TCN TGG or AAR GTN CAY GCN GGN TTY ACN TCN TCN TGG for Humicola
   5' primer for KVHK/AGFWSSW: AAR GTN CAY AAR GGN TTY TGG WSY WSY TGG or AAR GTN CAY GCN GGN TTY TGG WSY WSY TGG for P. camenbertii
   3' primer for GHSLGG/AA : NGC NSC NCC YAR NGA RTG NCC or NGC NSC NCC YAR RCT RTG NCC for all five sequences
Set 4)
   5' primer for GHSLGG/AA: GGN CAY TCN YTN GGN GSN GCN or GGN CAY AGY YTN GGN GSN GCN for all five sequences
   3' primer for PRVGN/D: RTY NCC NAC YCK NGG for all the genes except Absidia
   3' primer for TQGQPRVGN/D: RTY NCC NAC YCK NGG YTG NCC YTG NGT for Absidia
Set 5)
   5' primer for PRVGN/D: CCN MGR GTN GGN RAY for all the genes except Absidia
   5' primer for PRVGN/DPAFA: CCN MGR GTN GGN RAY CCN GCN TTY GCN for Absidia
   3' primer for RDIVPH/R/K: YK NGG NAC DAT RTC YCK for Absidia, Rhizopus and Rhizomucor sequences
   3' primer for I/FTHTRDIVPH/R/K: YK NGG NAC DAT RTC YCK NGT RTG NGT RAW for the two other sequences
Set 6)
   5' primer for RDIVPH/R/K: MGR GAY ATH GTN CCN MR for Absidia, Rhizopus and Rhizomucor sequences
   5' primer for RDIVPH/R/KLP: MGR GAY ATH GTN CCN MRN YTR CCN for the two other sequences
   3' primer for EYWIK/T: YKT DAT CCA RTA YTC for Rhizomucor, Humicola and P.camenbertii
   3' primer for PGVEYWIK/T: YKT DAT CCA RTA YTC NAC NCC NGG for Rhizopus
   3' primer for AGEEYWIK/T: YKT DAT CCA RTA YTC YTC NCC NGC for Absidia
Set 7)
   5' primer for EYWIK/T: GAR TAY TGG ATH AAR or GAR TAY TGG ATH ACN for Rhizomucor, Humicola and P.camenbertii
   5' primer for EYWIKSGT: GAR TAY TGG ATH AAR WSY GGN ACN for Rhizopus
   5' primer for EYWIKKDSS: GAR TAY TGG ATH AAR AAR GAY WSY WSY for Absidia
   3' primer for DHLSY: RTA NGA/RCT YAR RTG RTC for Absidia, Rhizopus and Rhizomucor sequences
   3' primer for IPDIPDHLSY: RTA NGA/RCT YAR RTG RTC NGG DAT RTC NGG DAT for Humicola
   3' primer for TDFEDHLSY: RTA NGA/RCT YAR RTG RTC YTC RAA RTC NGT for P.camenbertii

For the SOE-PCR the 5' primers from the first set of primers and the 3' primer for the last set of primers can be used.

The SOE-PCR fragments can then be combined with a lipase 5' and 3' end, when the 5' and 3' ends have been generated by PCR. The 5' end can be generated by PCR by using specific 5' primers (containing a sequence for the BamHI recognition site in the 5' end) for the 5' end of the genes of interest and using the complementary sequence from the 5' primer from the first set of primers as the 3' primer. The 3' end can be generated by PCR by using specific 3' primers (containing a sequence for the XbaI recognition site in the 5' end) for the 3' end of the genes of interest and the complementary sequence from the 3' primer from the last set of primers as the 5' primer.

A second SOE is then used to generate the complete sequence, by using the specific 5' and 3' primers from the genes of interest.

The genes can then be cloned into the yeast vector pJSO26 as a BamHI-XbaI fragment (see WO 97/07205).

### Example 3

The overall same method as described in example 2 can be used for amplification and recombination of PCR fragments of Pseudomonas lipases. The term "overall same method" denotes that it may be advantageous to use slightly different vectors as compared to example 2. Based on the sequence and primer information disclosed below it is a matter of routine for a person skilled in the art to modify the vectors etc. from example 2, in order to recombine below mentioned Pseudomonas lipases according to a shuffling method of the invention.

The Pseudomonas lipases mentioned below are aligned using the alignment program from Geneworks (using the following parameters:cost to open a gap = 5, cost to lengthen a gap = 25, Minimum Diagonal lLength = 4, Maximum Diagonal Length = 10, Consensus cutoff = 50%).

### Pseudomonas lipases

Pseudomonas aeruginosa TE3285 (file ate3285d)
Pseudomonas pseudoalcaligenes M1 (Lipomax wt) (file pseudm1d)
Pseudomonas sp. SD705 (mature)(file spsd705d)
Pseudomonas wisconsinensis (file wisconsd) Proteus vulgaris K80 (file provulgd) Pseudomonas fragi IFO 12049 (file fr12049d).

Suitable primers for shuffling of Pseudomonas lipases:
I = Inosin, Numbers refer to the numbers in the alignment(see figure 4), S means sense strand, the antisense oligonucleotide is of course also used:
109-131
   S1: 5'-TA(C/T)CCIAT(C/T)(G/T)I(C/T)T(G/A)(G/A)(C/T)ICA(C/T)GG-3'
250-269
   S2: 5'-GA(G/A)(G/C)IICGIGGIG(A/C)I(G/C)A(G/A)(T/C)T-3'
318-343
   S3: 5'-GT(C/A)AA(C/T)(C/T)T(G/A)ITCGG(C/T)CA(C/T)AG(C/T)CAIGG-3'
607-628 S4: 5'-TIAA(C/T) (G/C/A) (G/C/A) (C/T/A) (A/C) (A/G) I (T/C) (A/T) (C/T) CCI (C/T) (A /G) (T/G/A)GG-3'
801-817
   S5: 5'-AA(C/T)GA(C/T)GG(C/T)(C/A/T)TGGT(C/T/G)GG-3'
871-890 S6: 5'-CA(C/T) (C/G)T(C/G)GA(C/T) (G/A) (A/C/T) (G/C) (G/A)T(G/C/A)AACCA-3'

## Claims

1. A method for shuffling heterologous DNA sequences encoding an enzymatic activity or enzymatic activities of interest, the method comprising the following steps,
i) identifying one or more conserved region(s) in two or more of the heterologous sequences;
ii) constructing at least two sets of PCR primers directed to the conserved region(s), wherein the sequence regions between each set of PCR primers, including the primer sequences, have a homologous sequence overlap of at least 10 base pairs within the conserved region(s);
iii) performing two PCR amplification reactions with the at least two sets of PCR primers using the heterologous DNA sequences as templates;
iv) isolating and pooling PCR fragments generated in step iii) and performing a Sequence overlap extension PCR reaction (SOE-PCR) using said PCR fragments as templates; and
v) isolating PCR fragment(s) generated by the SOE-PCR reaction in step iv), wherein said isolated PCR fragment(s) comprise shuffled sequences and the partial DNA sequences originating from the homologous sequence overlaps in step ii) have at least 80% identity to one or more partial sequences in one or more of the original heterologous DNA sequences in step i).

2. The method according to claim 1 which comprises the following further steps:
vi) expressing recombinant proteins encoded by the isolated sequences from step v); and
vii) screening or selecting the recombinant proteins in a suitable screening or selection system for one or more recombinant protein(s) having a desired enzymatic activity.

3. The method according to claim 1 or 2, wherein the primers of step ii) have a homologous sequence overlap of at least 10 base pairs (bp) within the conserved region, preferably said homologous sequence overlap is at least 15 bp, more preferably at least 20 bp, and even more preferably at least 35 bp.

4. The method according to any of claims 1 - 3, wherein the enzymatic activity of interest is an amylase, lipase, cutinase, cellulase, oxidase, phytase, or protease activity.

5. The method according to any of claims 1 - 4, wherein the enzymatic activities are different enzymatic activities.

6. The method according to claim 5, wherein the enzymatic activity of interest is a serine protease, preferably a subtilase.

7. The method according to any of claims 1 - 6, wherein a conserved region of one heterologous sequence has at least 90% identity to a conserved region of another heterologous sequence; preferably at least 95% identity.

8. The method according to any of claims 1 - 7, wherein one heterologous DNA sequence comprises a partial sequence of at least 40 bp which does not exhibit a degree of identity of more than 50% with any partial sequence in another heterologous DNA sequence.

9. The method according to claim 8, wherein the first partial sequence is at least 60 bp, more preferably the first partial sequence is at least 80 bp, even more preferably the first partial sequence is at least 120 bp, and most preferably the first partial sequence is at least 500 bp.

## Patentansprüche

1. Verfahren zum Shuffling heterologer DNA-Sequenzen, kodierend eine enzymatische Aktivität oder enzymatische Aktivitäten von Interesse, wobei das Verfahren die folgenden Schritte umfasst,
i) Identifizieren einer oder mehrerer konservierter Region(en) in zwei oder mehreren der heterologen Sequenzen;
ii) Konstruieren mindestens zweier Sätze von PCR-Primern, die gegen die konservierte(n) Region(en) gerichtet sind, wobei die Sequenzregionen zwischen jedem Satz PCR-Primern, einschließlich der Primer-Sequenzen, eine homologe Sequenzüberlappung von mindestens 10 Basenpaaren innerhalb der konservierten Region(en) haben;
iii) Durchführen von zwei PCR-Vervielfältigungsreaktionen mit den mindestens zwei Sätzen PCR-Primern unter Verwendung der heterologen DNA-Sequenzen als Matrizen;
iv) Isolieren und Vereinen der in Schritt iii) generierten PCR-Fragmente und Durchführen einer Sequenzüberlappungsverlängerungs-PCR-Reaktion (Sequence overlap extension PCR reaction, SOE-PCR) unter Verwendung der PCR-Fragmente als Matrizen; und
v) Isolieren des/der durch die SOE-PCR-Reaktion in Schritt iv) generierten PCR-Fragments/Fragmente, wobei das/die isolierte(n) PCR-Fragment(e) geshuffelte Sequenzen umfassen, und die aus den homologen Sequenzüberlappungen in Schritt ii) stammenden DNA-Teilsequenzen mindestens 80% Identität zu einer oder mehreren Teilsequenzen in einer oder mehreren der ursprünglichen heterologen DNA-Sequenzen in Schritt i) haben.

2. Verfahren nach Anspruch 1, welches die folgenden weiteren Schritte umfasst:
vi) Exprimieren rekombinanter Proteine, die durch die isolierten Sequenzen aus Schritt v) kodiert sind; und
vii) Durchmustern oder Auswählen der rekombinanten Proteine in einem geeigneten Durchmusterungs- oder Auswahlsystems für ein oder mehrere rekombinante(s) Protein(e) mit einer gewünschten enzymatischen Aktivität.

3. Verfahren nach Anspruch 1 oder 2, wobei die Primer von Schritt ii) eine homologe Sequenzüberlappung von mindestens 10 Basenpaaren (bp) innerhalb der konservierten Region haben, vorzugsweise wobei die homologe Sequenzüberlappung mindestens 15 bp, stärker bevorzugt mindestens 20 bp, und noch stärker bevorzugt mindestens 35 bp beträgt.

4. Verfahren nach einem beliebigen der Ansprüche 1 - 3, wobei die enzymatische Aktivität von Interesse eine Amylase-, Lipase-, Cutinase-, Cellulase-, Oxidase-, Phytase- oder Proteaseaktivität ist.

5. Verfahren nach einem beliebigen der Ansprüche 1 - 4, wobei die enzymatischen Aktivitäten unterschiedliche enzymatische Aktivitäten sind.

6. Verfahren nach Anspruch 5, wobei die enzymatische Aktivität von Interesse eine Serinprotease, vorzugsweise eine Subtilase ist.

7. Verfahren nach einem beliebigen der Ansprüche 1 - 6, wobei eine konservierte Region einer heterologen Sequenz mindestens 90 % Identität zu einer konservierten Region einer anderen heterologen Sequenz hat; vorzugsweise mindestens 95 % Identität.

8. Verfahren nach einem beliebigen der Ansprüche 1 - 7, wobei eine heterologe DNA-Sequenz eine Teilsequenz von mindestens 40 bp umfasst, die nicht einen Identitätsgrad von mehr als 50 % mit einer beliebigen Teilsequenz in einer anderen heterologen DNA-Sequenz aufweist.

9. Verfahren nach Anspruch 8, wobei die erste Teilsequenz mindestens 60 bp beträgt, stärker bevorzugt wobei die erste Teilsequenz mindestens 80 bp beträgt, noch stärker bevorzugt wobei die erste Teilsequenz mindestens 120 bp beträgt, und am stärksten bevorzugt wobei die erste Teilsequenz mindestens 500 bp beträgt.

## Revendications

1. Méthode de recombinaison de séquences d'ADN hétérologues codant une activité enzymatique ou des activités enzymatiques d'intérêt, la méthode comprenant les étapes suivantes,
i) identifier une ou plusieurs régions conservées dans deux ou plus des séquences hétérologues ;
ii) construire au moins deux jeux d'amorces de PCR dirigées vers la ou les régions conservées, dans lesquels les régions de séquence entre chaque jeu d'amorces de PCR, incluant les séquences de l'amorce, ont un chevauchement de séquence homologue d'au moins 10 paires de bases au sein de la ou des régions conservées ;
iii) réaliser deux réactions d'amplification PCR avec les au moins deux jeux d'amorces de PCR en utilisant les séquences d'ADN hétérologues comme matrices ;
iv) isoler et mettre en commun les fragments de PCR générés à l'étape iii) et réaliser une réaction d'extension PCR par chevauchement de séquence (SOE-PCR) en utilisant lesdits fragments de PCR comme matrices ; et
v) isoler le ou les fragments PCR générés par la réaction SOE-PCR de l'étape iv), dans laquelle ledit ou lesdits fragments PCR isolés comprennent des séquences aléatoires et les séquences d'ADN partielles provenant des chevauchements de séquence homologue de l'étape ii) ont au moins 80% d'identité avec une ou plusieurs séquences partielles de l'une ou plusieurs des séquences d'ADN hétérologue originales de l'étape i).

2. Méthode selon la revendication 1 comprenant les étapes additionnelles suivantes :
vi) exprimer les protéines recombinantes codées par les séquences isolées de l'étape v) ; et
vii) cribler ou sélectionner les protéines recombinantes dans un criblage ou un système de sélection approprié pour une ou plusieurs protéines recombinantes ayant une activité enzymatique désirée.

3. Méthode selon la revendication 1 ou 2, dans laquelle les amorces de l'étape ii) ont un chevauchement de séquence homologue d'au moins 10 paires de bases (pb) dans la région conservée, de préférence ledit chevauchement de séquence homologue est d'au moins 15 pb, préférentiellement d'au moins 20 pb, et encore plus préférentiellement d'au moins 35 pb.

4. Méthode selon l'une quelconque des revendications 1-3 , dans laquelle l'activité enzymatique d'intérêt est une activité amylase, lipase, cutinase, cellulase, oxydase, phytase ou protéase.

5. Méthode selon l'une quelconque des revendications 1-4, dans laquelle les activités enzymatiques sont des activités enzymatiques différentes.

6. Méthode selon la revendication 5, dans laquelle l'activité enzymatique d'intérêt est une sérine protéase, de préférence une subtilase.

7. Méthode selon l'une quelconque des revendications 1-6, dans laquelle une région conservée d'une séquence hétérologue a au moins 90% d'identité avec une région conservée d'une autre séquence hétérologue, de préférence au moins 95% d'identité.

8. Méthode selon l'une quelconque des revendications 1-7, dans laquelle une séquence d'ADN hétérologue comprend une séquence partielle d'au moins 40 pb qui ne présente pas un degré d'identité de plus de 50% avec une séquence partielle quelconque dans une autre séquence d'ADN hétérologue.

9. Méthode selon la revendication 8, dans laquelle la première séquence partielle est d'au moins 60 pb, de préférence la première séquence partielle est d'au moins 80 pb, préférentiellement la première séquence partielle est d'au moins 120 pb, et de façon hautement préférée la première séquence partielle est d'au moins 500 pb.
